# EUROPÄISCHE PATENTANMELDUNG

(11) **EP 3 175 871 A1**
(43) Veröffentlichungstag der Anmeldung: **07.06.2017**
(21) Anmeldenummer: 16002524.3
(22) Anmeldetag: 28.11.2016
(51) Int. Cl.: A61M 1/00, A61F 13/00, A61F 13/02

(54) **ANORDNUNG ZUM ENTFERNEN VON WUNDSEKRET AUS KÖRPERHÖHLEN**

(30) Priorität: 03.12.2015 DE 102015015559
(71) Anmelder: Primed Halberstadt Medizintechnik GmbH, 38820 Halberstadt (DE)
(72) Erfinder: Leibitzki, Sascha, 38889 Blankenburg (DE); Süß, Steffen, 38820 Halberstadt (DE)
(74) Vertreter: Fischer, Volker

(57) **Zusammenfassung**

Die Erfindung betrifft eine Anordnung zum Entfernen von Wundsekret aus Körperhöhlen. Sie betrifft insbesondere eine Anordnung zur Unterdruck-Wundtherapie unter Verwendung einer Saugpumpe (4) zur Erzeugung eines Unterdruckes und zur Absaugung des Wundsekretes.

Die Anordnung umfasst zumindest
- einen in die Körperhöhle eingebrachten, mit einem Drainageschlauch (2) konnektierten ersten Körper (1) aus sekretaufnehmendem Material,
- eine Saugpumpe (4) zum Absaugen des Wundsekretes mittels einer Saugleitung (5) sowie
- eine Adaptereinheit (3), an die einerseits das aus der Körperhöhle geführte Ende des Drainageschlauches (2) und andererseits die Saugleitung (5) und, sofern die Saugpumpe (4) auch eine Messleitung umfasst, auch die Messleitung angeschlossen sind.

Dabei umfasst die Adaptereinheit (3) einen in einer luftdichten Hülle (8) befindlichen zweiten Körper (7) aus sekretaufnehmendem Material und mindestens einen Anschluss (10, 11) für den Drainageschlauch (2). Mindestens eine Innenseite von sich gegenüberliegenden, dem zweiten Körper (7) aus sekretaufnehmendem Material zugewandten Innenseiten der Hülle (8) der Adaptereinheit (3) weist eine Strukturierung (9) auf. Die Hülle (8) der Adaptereinheit (3) weist weiterhin einen Bereich zur individuellen Einbringung einer Öffnung (12) auf. Die jeweils offenen Enden der Saugleitung (5) und ggf. der Messleitung sind in bzw. über einer individuell in den vorgenannten Bereich der Hülle (8) eingebrachten Öffnung (12) angeordnet, derart, dass die Saugleitung (5) und ggf. die Messleitung mit ihrem jeweils offenen Ende mit dem in der luftdichten Hülle (8) befindlichen zweiten Körper (7) aus sekretaufnehmendem Material wirkverbunden ist/sind, wobei eine Anbindung der Saugleitung (5) und ggf. der Messleitung an die in die Hülle (8) eingebrachte Öffnung (12) durch luftundurchlässiges Verkleben der Saugleitung (5) und ggf. der Messleitung mit der Außenseite der Hülle (8) der Adaptereinheit (3) erfolgt.

## Beschreibung

Die Erfindung betrifft eine Anordnung zum Entfernen von Wundsekret aus Körperhöhlen. Die Erfindung betrifft insbesondere eine Anordnung zur Unterdruck-Wundtherapie, in der medizinischen Praxis auch als Vakuumtherapie bezeichnet, unter Verwendung einer Saugpumpe zur Erzeugung eines Unterdruckes und zur Absaugung des Wundsekretes.

Wundsekrete sind zumeist wässrige Absonderungen des Körpers infolge einer Verletzung, die aus einer Wunde austreten können. Zur Vermeidung von Entzündungen und ggf. Abszessen ist es erforderlich, Wundsekrete aus einer Wunde zu entfernen. Letztlich wird dadurch auch eine Verbesserung der Wundheilung erreicht.

Eine in der medizinischen Praxis etablierte Methode zur Entfernung von Wundsekret ist die Unterdruck-Wundtherapie. Dabei wird ein zumeist luftdichter Wundverschluss realisiert und das Wundsekret über eine mit einer Einrichtung zur Erzeugung eines Unterdruckes wirkverbundene Drainage abgesaugt. Neben der verbesserten Wundheilung durch die Entfernung des Wundsekretes kommen bei der Unterdruck-Wundtherapie zusätzlich Vorteile eines auf das Gewebe wirkenden Unterdruckes zur Beschleunigung der Wundheilung und zur Wundverkleinerung zum Tragen. Um eine möglichst vollständige Ableitung des Wundsekrets zu erreichen, ist es bei der Unterdruck-Wundtherapie üblich, der Form der Wunde angepasste Körper aus sekretaufnehmendem Material, meist in Form eines Schaumstoffkörpers, so in die Wunde einzubringen, dass diese den Unterdruck in alle Bereiche der Wunde vermitteln und das Wundsekret aus allen Bereichen der Wunde aufnehmen. Das Wundsekret wird über einen mit dem Körper aus sekretaufnehmendem Material konnektierten Drainageschlauch aus dem Körper abgesaugt. Dabei wird unter Konnektieren entweder ein Einstecken des Drainageschlauches bzw. einer Saugleitung einer Saugpumpe in den Körper aus sekretaufnehmendem Material oder das Aufkleben eines Portes, der mit dem proximalen Ende des Drainageschlauches bzw. einer Saugleitung einer Saugpumpe verbunden ist, auf den Körper aus sekretaufnehmendem Material verstanden. Ein entsprechender Körper aus sekretaufnehmendem Material in Form eines Schaumstoffkörpers wird beispielsweise in der DE 10 2004 062 877 A1 beschrieben. Der Körper aus sekretaufnehmendem Material verbleibt eine gewisse Zeit in der Wunde, muss jedoch bei fortschreitendem Heilungsprozess aus der Wunde entfernt bzw. gewechselt werden. Oft wird die Unterdruck-Wundtherapie so über mehrere Zyklen mit jeweils unterschiedlichen Körpern aus sekretaufnehmendem Material angewandt.

Saugpumpen zum Absaugen von Wundsekret werden von verschiedenen Herstellern angeboten. Je nach Hersteller bzw. Ausführung der Saugpumpe verfügen die Saugpumpen über ein oder mehrere unterschiedlich ausgebildete Saug-, Spül- und Messleitungen, die zur ordnungsgemäßen Funktion der Saugpumpe mit der Wunde bzw. dem Körper aus sekretaufnehmendem Material konnektiert sein müssen. Zumeist verwendet jeder Hersteller dazu ein spezielles, eigenes Anschlusssystem. Dies führt dazu, dass in der Praxis Komponenten für eine Anordnung zur Unterdruck-Wunddrainage von unterschiedlichen Herstellern bzw. auch unterschiedliche Ausführungen eines Herstellers nicht miteinander kombiniert werden können.

Ein weiteres erhebliches Problem besteht darin, dass Saugpumpen, die mindestens über eine Saug- und eine Messleitung verfügen, stets so mit der Wunde verbunden sein müssen, dass über die Saugleitung das Wundsekret eingesaugt werden kann und über die Messleitung der durch die Saugpumpe in der Wunde erzeugte Unterdruck zur Saugpumpe weitergeleitet wird, um über die Saugleistung der Pumpe einen vorbestimmten Unterdruck in der Wunde zu steuern. Der Anschluss eines mit einem "normalen", einlumigen Drainageschlauch konnektierten Körpers aus sekretaufnehmendem Material an eine solche Saugpumpe ist nicht möglich, weil das zur Steuerung der Saugpumpe notwendige Signal über den in der Wunde erzeugten Unterdruck nicht zur Saugpumpe übertragen wird und somit keine Steuerung der Saugpumpe erfolgen kann. Dabei ist es letztlich unerheblich, ob, wie bei einigen Herstellern üblich, die Saug- und die Messleitung in einem Schlauch mit zwei Lumen zusammengefasst sind oder, wie von anderen Herstellern angeboten, Saug- und Messleitung als zwei getrennte Schläuche ausgeführt sind.

Aufgabe der vorliegenden Erfindung ist es daher, eine Anordnung zum Entfernen von Wundsekret aus Körperhöhlen bereitzustellen, welche die vorgenannten Probleme des Standes der Technik überwindet. Insbesondere ist es die Aufgabe der Erfindung, eine Anordnung bereitzustellen, die es ermöglicht, unterschiedliche Komponenten für die Ausbildung einer Unterdruck-Wunddrainage zu verwenden, und zwar unabhängig davon, mit welcher Art von Drainageschlauch, ein-, zwei- oder mehrlumig, der in die Wunde einbringbare Körper aus sekretaufnehmendem Material zur Aufnahme des Wundsekretes konnektiert ist und wie der Anschluss des Drainageschlauches der Saugpumpe ausgebildet ist.

Die Aufgabe wird durch eine Anordnung zum Entfernen von Wundsekret mit den Merkmalen des Hauptanspruches gelöst. Vorteilhafte Ausgestaltungen sind in den abhängigen Ansprüchen angegeben.

Eine Anordnung zum Entfernen von Wundsekret aus Körperhöhlen umfasst zumindest
- einen in die Körperhöhle eingebrachten, gegenüber der äußeren Umgebung der Körperhöhle luftdicht verschlossenen mit einem Drainageschlauch konnektierten ersten Körper aus sekretaufnehmendem Material,
- eine Saugpumpe zur Erzeugung eines Unterdruckes und zum Absaugen des Wundsekretes mittels einer Saugleitung sowie
- eine Adaptereinheit, an die einerseits das aus der Körperhöhle geführte Ende des Drainageschlauches und andererseits die Saugleitung und, sofern die Saugpumpe auch eine Messleitung umfasst, auch die Messleitung angeschlossen sind.

Dabei umfasst die Adaptereinheit einen in einer luftdichten Hülle befindlichen zweiten Körper aus sekretaufnehmendem Material und mindestens einen Anschluss für den Drainageschlauch. Mindestens eine Innenseite von sich gegenüberliegenden, dem zweiten Körper aus sekretaufnehmendem Material zugewandten Innenseiten der Hülle der Adaptereinheit weist eine Strukturierung auf, die bei Unterdruck innerhalb der Hülle der Adaptereinheit ein luft- und/oder flüssigkeitsundurchlässiges Aneinanderkleben der sich gegenüberliegenden Innenseiten der Hülle verhindert. Die Hülle der Adaptereinheit weist weiterhin einen Bereich zur individuellen Einbringung einer Öffnung auf. Die jeweils offenen Enden der Saugleitung und ggf. der Messleitung sind in bzw. über einer individuell in den vorgenannten Bereich der Hülle eingebrachten Öffnung angeordnet, derart, dass die Saugleitung und ggf. die Messleitung mit ihrem jeweils offenen Ende mit dem in der luftdichten Hülle befindlichen zweiten Körper aus sekretaufnehmendem Material wirkverbunden ist/sind, wobei eine Anbindung der Saugleitung und ggf. der Messleitung an die in die Hülle eingebrachte Öffnung durch luftundurchlässiges Verkleben der Saugleitung und ggf. der Messleitung mit der Außenseite der Hülle der Adaptereinheit erfolgt.

Die erfindungsgemäße Anordnung ermöglicht, einen in eine Körperhöhle einzubringenden ersten Körper aus sekretaufnehmendem Material individuell an die Körperhöhle anzupassen, wobei nur ein einlumiger Drainageschlauch aus der Körperhöhle herauszuführen ist, der dann mit der Adaptereinheit zu verbinden ist. Dies hat den besonderen Vorteil, dass bei der Ausbildung des in die Körperhöhle einzubringenden ersten Körpers aus sekretaufnehmendem Material ausschließlich Bedingungen, die durch die Körperhöhle vorgegeben werden, berücksichtigt werden müssen. Spezielle Anschlussbedingen einer Saugpumpe beispielsweise brauchen bei der Ausbildung des in die Körperhöhle einzubringenden ersten Körpers aus sekretaufnhemendem Material nicht beachtet werden. Hierdurch ist eine besonders zweckmäßige Anpassung des ersten Körpers aus sekretaufnehmendem Material an die Bedingungen der Körperhöhle möglich. Durch die Verbindung der erfindungsgemäßen Adaptereinheit über den Drainageschlauch mit dem in die Körperhöhle eingebrachten ersten Körper aus sekretaufnehmendem Material wird erreicht, dass innerhalb der Adaptereinheit, welche einen zweiten Körper aus sekretaufnehmendem Material beinhaltet, die gleichen Bedingungen herrschen wie innerhalb der Körperhöhle. Der zweite Körper aus sekretaufnehmendem Material in der Adaptereinheit ist über den einlumigen Drainageschlauch mit dem in die Körperhöhle eingebrachten ersten Körper aus sekretaufnehmendem Material wirkverbunden. An die Adaptereinheit können dann unterschiedlichste medizinische Saugpumpen angeschlossen werden.

Der bei der Unterdruck-Wundtherapie üblicherweise eingesetzte, der Form der Wunde angepasste Körper aus sekretaufnehmendem Material wird erfindungsgemäß in zwei Teilkörper aufgeteilt, wobei der erste Teilkörper oder erste Körper aus sekretaufnehmendem Material in der Wunde luftdicht eingeschlossen und der zweite Teilkörper oder Körper aus sekretaufnehmendem Material in der erfindungsgemäßen Adaptereinheit angeordnet ist.

Der in der luftdichten Hülle der Adaptereinheit angeordnete zweite Körper aus sekretaufnehmendem Material ist bevorzugt als Schaumstoffkörper mit einer Porosität von 30 bis 60 ppi ausgebildet.

Weiterhin bevorzugt ist der zweite Körper aus sekretaufnehmendem Material quaderförmig ausgebildet, wobei die Größe der Fläche zweier sich gegenüberliegender Hauptseitenflächen des Quaders erheblich größer ist als die Größe der Fläche der übrigen Nebenseitenflächen des Quaders. Besonders bevorzugt ist die Größe der Fläche der Hauptseitenflächen des Quaders mindestens 5 x größer als die Größe der Fläche der Nebenseitenflächen des Quaders.

Ebenfalls bevorzugt ist die Hülle der Adaptereinheit aus einer luftundurchlässigen Folie ausgebildet. Die Strukturierung zur Vermeidung des Aneinanderklebens der sich gegenüberliegenden Innenseiten der Hülle bei Unterdruck innerhalb der Hülle der Adaptereinheit ist bevorzugt an mindestens einer der den Hauptseitenflächen des Quaders zugewandten Innenflächen der Folie ausgebildet. Bevorzugt ist die Strukturierung in Form von aneinanderliegenden parallelen im Querschnitt dreieckförmigen Rippen mit einer Höhe zwischen 1 und 3 mm ausgebildet.

Weiterhin bevorzugt ist in der Hülle der Adaptereinheit im Bereich einer der Nebenseitenflächen des quaderförmigen zweiten Körpers aus sekretaufnehmendem Material ein in die Hülle mündender Schlauch mit einem am der Hülle abgewandten Schlauchende angeordneten Stufenverbinder zum Anschluss des Drainageschlauches angeordnet.

In der Hülle der Adaptereinheit ist im Bereich einer Hauptseitenfläche des quaderförmigen zweiten Körpers aus sekretaufnehmendem Material ein Bereich zur individuellen Einbringung einer Öffnung angeordnet. Der Bereich zur individuellen Einbringung einer Öffnung in die Hülle der Adaptereinheit ist bevorzugt als ein mittels Schere oder Skalpell durchschneidbarer Bereich ausgebildet.

Des Weiteren bevorzugt ist die Außenseite der Hülle der Adaptereinheit an der Seite, die der Seite, in der der Bereich zur individuellen Einbringung einer Öffnung angeordnet ist, gegenüberliegt, mindestens bereichsweise mit einer hautverträglichen Klebebeschichtung versehen.

Die Erfindung soll nachfolgend anhand eines Ausführungsbeispieles näher erläutert werden. Die zugehörigen Zeichnungen zeigen in
- Fig. 1:: Anordnung zum Entfernen von Wundsekret aus einer Bauchwunde, in
- Fig. 2a:: eine Adaptereinheit mit aufgeklebtem Port einer Saugleitung einer Saugpumpe und in
- Fig. 2b:: einen Schnitt durch eine Adaptereinheit mit aufgeklebtem Port einer Saugleitung.

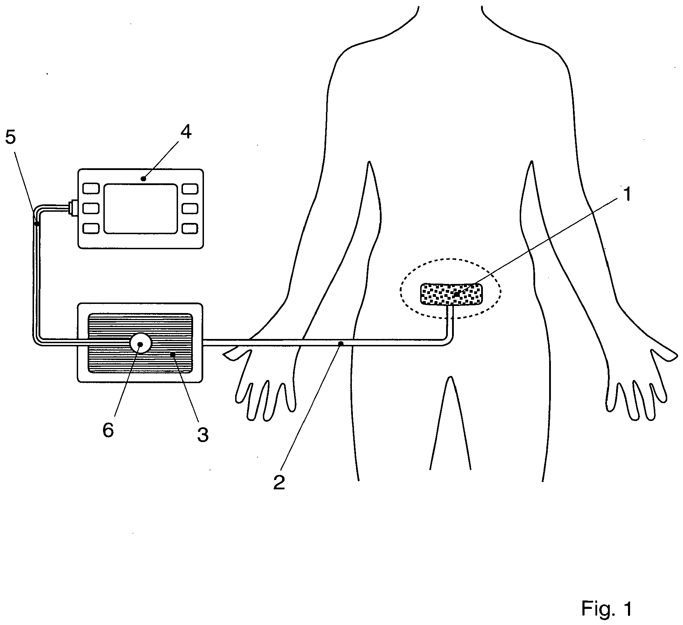

Fig. 1 zeigt einen in eine Bauchwunde eingebrachten ersten Körper 1 aus sekretaufnehmendem Material in Form eines dem Hohlraum der Wunde angepassten ersten Schaumstoffkörpers 1. In den ersten Schaumstoffkörper 1 eingesteckt und so mit dem ersten Schaumstoffkörper 1 konnektiert ist ein einlumiger Drainageschlauch 2. Der Drainageschlauch 2 ist aus der Wunde herausgeführt. Die Wunde wurde nach dem Einbringen des ersten Schaumstoffkörpers 1 und dem Herausführen des Drainageschlauches 2 durch Überkleben mit einer luftundurchlässigen Folie gegenüber der äußeren Umgebung der Wunde luftdicht verschlossen. Der Drainageschlauch 2 ist mit einer Adaptereinheit 3 verbunden. Fig. 1 zeigt weiterhin eine Saugpumpe 4 mit einer Saugleitung 5, an deren der Saugpumpe 4 abgewandtem Ende ein Port 6 angebracht ist. Die Saugleitung 5 ist zweilumig ausgebildet. Sie umfasst ein Sauglumen und ein Messlumen. Beide Lumen sind mit der Adaptereinheit 3 verbunden.

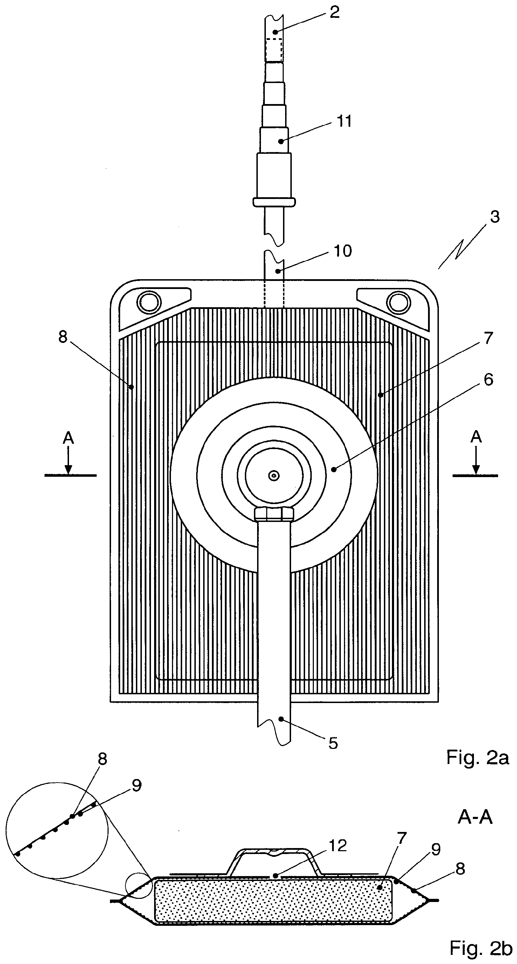

Die Fig. 2a und 2b zeigen die Adaptereinheit 3. Die Adaptereinheit 3 umfasst einen quaderförmigen zweiten Körper 7 aus sekretaufnehmendem Material. Der zweite Körper 7 ist als zweiter Schaumstoffkörper mit einer Porosität von ca. 40 bis 45 ppi ausgebildet. Der zweite Körper 7 ist in eine Hülle 8 aus einer luftdichten Folie eingebettet. Die Hülle 8 wird von zwei Folienzuschnitten gebildet, die an ihren Randbereichen luftdicht miteinander verschweißt sind. In dem so gebildeten Inneraum der Hülle 8 befindet sich der zweite Körper 7. Die dem zweiten Körper 7 zugewandten Innenseiten der Hülle 8 sind mit parallel zueinander liegenden, sich über die gesamte Länge der Innenseiten erstreckenden dreieckförmigen Erhebungen 9 strukturiert. Diese Strukturierung der Innenseiten verhindert ein Aneinanderkleben der sich gegenüberliegenden Innenseiten der Hülle 8 im Falle eines erheblichen Unterdruckes im Inneren der Hülle 8. In der Verweißung der Folienzuschnitte im Bereich einer Stirnseite des quaderförmigen zweiten Körpers 7 ist ein in das Innere der Hülle 8 ragender einlumiger Schlauch 10 angeordnet, an dessen offenem Ende sich ein Stufenverbinder 11 befindet. Auf den Stufenverbinder 11 ist der aus der Wunde kommende mit dem Schaumstoffkörper 1 in der Wunde konnektierte Drainageschlauch 2 aufgesteckt. Fig. 2b zeigt weiterhin eine in die Hülle 8 individuell eingebrachte Öffnung 12. Diese Öffnung 12 überdeckend, ist auf die Außenseite der Hülle 8 der Port 6 der Saugleitung 5 aufgeklebt, und zwar derart, dass beide Lumen der Saugleitung 5 mit dem Innenraum der Hülle 7 wirkverbunden sind.

Bestimmungsgemäß wird durch die Saugpumpe 4 ein über die Saugleitung 5 in den Innenraum der Adaptereinheit 3 zugeführter vorbestimmter Unterdruck erzeugt. Über das Messlumen der Saugleitung 5 wird der im Innenraum der Adaptereinheit 3 erzeugte Unterdruck der Saugpumpe 4 als Rückkopplungsgröße zur Regelung der Saugleistung der Saugpumpe 4 zugeleitet. Der Unterdruck im Innenraum der Adaptereinheit 3 kann somit geregelt werden. Über den einlumigen Schlauch 10 und die Drainageleitung 2 wird der im Innenraum der Adaptereinheit 3 herrschende Unterdruck zu dem im Hohlraum der Wunde platzierten ersten Schaumstoffkörper 1 übertragen, wodurch in der Wunde ein vorbestimmter Unterdruck erzeugt wird. Dadurch werden zum einen im ersten Schaumstoffkörper 1 bzw. der Wunde angesammeltes Sekret und ggf. auch Gase über die Drainageleitung 2 zunächst in den zweiten Körper 7 in der Adaptereinheit 3 und nachfolgend aus diesem zweiten Körper 7 über das Sauglumen der Saugleitung 5 in die Saugpumpe abgesaugt und zum anderen ein auf das Wundgewebe wirkender, die Wundheilung befördernder sowie den Wundraum verkleinernder vorbestimmt geregelter Unterdruck realisiert.
Der besondere Vorteil der beschriebenen Anordnung besteht darin, dass einerseits an die Adaptereinheit 3 nahezu beliebige medizinische Saugpumpen 4 anschließbar sind und andererseits der in die Wunde einzubringende erste Schaumstoffkörper 1 optimal an den Wundraum angepasst werden kann.

### Liste der verwendeten Bezugszeichen

1 - erster Körper aus sekretaufnehmendem Material / erster Schaumstoffkörper
2 - Drainageschlauch
3 - Adaptereinheit
4 - Saugpumpe
5 - Saugleitung
6 - Port
7 - zweiter Körper aus sekretaufnehmenden Material / zweiter Schaumstoffkörper
8 - Hülle
9 - Erhebung
10 - Schlauch
11 - Stufenverbinder
12 - Öffnung

## Patentansprüche

1. Anordnung zum Entfernen von Wundsekret aus Körperhöhlen, zumindest umfassend
- einen in die Körperhöhle eingebrachten, gegenüber der äußeren Umgebung der Körperhöhle luftdicht verschlossenen, mit einem Drainageschlauch (2) konnektierten ersten Körper (1) aus sekretaufnehmendem Material,
- eine Saugpumpe (4) zur Erzeugung eines Unterdruckes und zum Absaugen des Wundsekretes mittels einer Saugleitung (5) sowie
- eine Adaptereinheit (3), an die einerseits das aus der Körperhöhle ragende Ende des Drainageschlauches (2) und andererseits die Saugleitung (5) und, sofern die Saugpumpe (4) auch eine Messleitung umfasst, auch die Messleitung angeschlossen sind,
wobei die Adaptereinheit (3) einen in einer luftdichten Hülle (8) befindlichen zweiten Körper (7) aus sekretaufnehmendem Material und mindestens einen Anschluss (10, 11) für den Drainageschlauch (2) umfasst, mindestens jeweils eine Innenseite von sich gegenüberliegenden, dem zweiten Körper (7) aus sekretaufnehmendem Material zugewandten Innenseiten eine Strukturierung (9) aufweist, die bei Unterdruck innerhalb der Hülle (8) der Adaptereinheit (3) ein luft- und/oder flüssigkeitsundurchlässiges Aneinanderkleben sich gegenüberliegender Innenseiten verhindert, ein Bereich der Hülle (8) zur individuellen Einbringung einer Öffnung (12) ausgebildet ist und die Saugleitung (5) und ggf. die Messleitung über eine individuell in den vorgenannten Bereich der Hülle (8) eingebrachte Öffnung (12) an die Adaptereinheit (3) angeschlossen ist/sind, derart, dass die Saugleitung (5) und ggf. die Messleitung mit ihrem jeweils offenen Ende mit dem in der Adaptereinheit (3) befindlichen zweiten Körper (7) aus sekretaufnehmendem Material wirkverbunden ist/sind und die Anbindung der Saugleitung (5) und ggf. der Messleitung an die in die Hülle (8) eingebrachte Öffnung (12) durch luftundurchlässiges Verkleben der Saugleitung (5) und ggf. der Messleitung mit der Außenseite der Hülle (8) der Adaptereinheit (3) erfolgt.

2. Anordnung nach Anspruch 1, **dadurch gekennzeichnet, dass** der zweite Körper (7) aus sekretaufnehmendem Material als Schaumstoffkörper (7) mit einer Porosität von 30 bis 60 ppi ausgebildet ist.

3. Anordnung nach Anspruch 1 oder 2, **dadurch gekennzeichnet, dass** der zweite Körper (7) aus sekretaufnehmendem Material quaderförmig ausgebildet ist, wobei die Größe der Fläche zweier sich gegenüberliegender Hauptseitenflächen des Quaders (7) erheblich größer ist als die Größe der Fläche der übrigen Nebenseitenflächen des Quaders.

4. Anordnung nach Anspruch 3, **dadurch gekennzeichnet, dass** die Fläche der Hauptseitenflächen des Quaders (7) mindestens 5 x größer ist als die Fläche der Nebenseitenflächen des Quaders (7).

5. Anordnung nach einem der vorhergehenden Ansprüche, **dadurch gekennzeichnet, dass** die Hülle (8) der Adaptereinheit (3) aus einer luftundurchlässigen Folie ausgebildet ist.

6. Anordnung nach einem der Ansprüche 3 bis 5, **dadurch gekennzeichnet, dass** die Strukturierung auf mindestens einer der den Hauptseitenflächen des Quaders (7) zugewandten Innenflächen der Folie ausgebildet ist.

7. Anordnung nach einem der vorhergehenden Ansprüche, **dadurch gekennzeichnet, dass** die Strukturierung in Form von aneinanderliegenden parallelen, im Querschnitt dreieckförmigen Rippen (9) mit einer Höhe zwischen 1 und 3 mm ausgebildet ist.

8. Anordnung nach einem der Ansprüche 3 bis 7, **dadurch gekennzeichnet, dass** der Anschluss (10, 11) für den Drainageschlauch (2) in der Hülle (8) im Bereich einer der Nebenseitenflächen des zweiten Körpers (7) aus sekretaufnehmendem Material angeordnet ist und der Anschluss (10, 11) als ein in die Hülle (8) mündender Schlauch (10) mit einem am der Hülle (8) abgewandten Schlauchende angeordneten Stufenverbinder (11) ausgebildet ist.

9. Anordnung nach einem der Ansprüche 3 bis 8, **dadurch gekennzeichnet, dass** der Bereich der Hülle (8) zur individuellen Einbringung einer Öffnung (12) im Bereich einer der Hauptseitenflächen des zweiten Körpers (7) aus sekretaufnehmendem Material angeordnet und als ein mittels Schere oder Skalpell durchschneidbarer Bereich ausgebildet ist.

10. Anordnung nach einem der vorhergehenden Ansprüche, **dadurch gekennzeichnet, dass** die Außenseite der Hülle (8) an der Seite des zweiten Körpers (7) aus sekretaufnehmendem Material, die der Seite, in der der Bereich zur individuellen Einbringung einer Öffnung (12) angeordnet ist, gegenüberliegt, mindestens bereichsweise eine hautverträgliche Klebebeschichtung aufweist.
